(19) Europäisches Patentamt
European Patent Office
Office européen des brevets

(11) **EP 1 939 167 A1**

(12) **EUROPEAN PATENT APPLICATION**

(43) Date of publication:
**02.07.2008 Bulletin 2008/27**

(21) Application number: **06026328.2**

(22) Date of filing: **19.12.2006**

(51) Int Cl.:
*C07C 51/41* (2006.01)    *C07C 55/02* (2006.01)
*C07C 55/16* (2006.01)    *C07C 55/18* (2006.01)
*C08K 5/098* (2006.01)    *C08L 23/12* (2006.01)

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(71) Applicant: **Borealis Technology Oy**
**06201 Porvoo (FI)**

(72) Inventors:
• **Wolfschwenger, Johannes**
  **4491 Niederneukirchen (AT)**
• **Gahleitner, Markus**
  **4501 Neuhofen/Krems (AT)**

(74) Representative: **Grünecker, Kinkeldey,**
**Stockmair & Schwanhäusser**
**Anwaltssozietät**
**Leopoldstrasse 4**
**80802 München (DE)**

(54) **Beta-nucleating agent for polyproplyene and process for its preparation**

(57)    The present invention relates to a novel β-nucleating agent for polypropylene and a process for preparing same. Preferably, the β-nucleating agent is a water-free admixture of calcium carbonate and a calcium salt of a dibasic organic acid.

EP 1 939 167 A1

## Description

The present invention concerns a novel method for preparing a β-nucleating agent for polypropylene, a novel β-nucleating agent obtained thereby and a polypropylene composition comprising this β-nucleating agent.

## Prior art

Polypropylene crystallizes typically when cooling down a melt in the monoclinic α-modification. In addition to this α-modification, polypropylene, however, may also crystallize in the hexagonal β-modification and the orthorhombic γ-modification. The β-modification is characterized by improved mechanical properties, in particular improved impact strength and improved resistance to stress cracking.

Typically, crystallization in the β-modification is achieved by adding specific β-nucleating agents, such as quinacridone pigments, which are disclosed in EP 0 177 961 A2. A further well-known class of β-nucleating agents are Group II salts of dibasic organic acids.

US-A-5,231,126 discloses that β-nucleation can be achieved by the admixing of isotactic polypropylene with a two-component β-nucleating agent consisting of a mixture of a dibasic acid with an oxide, hydroxide or acid salt of a Group II metal. Suitable examples of dibasic acids are pimelic acid, azelaic acid, o-phtalic acid, terephthalic acid and isophthalic acid and the like. Suitable oxide, hydroxides or acid salts of Group II metals are compounds comprising magnesium, calcium, strontium or barium, with typical examples including calcium carbonate or other carbonates.

A drawback of the two-component β-nucleating agent disclosed in this prior art document, however, is the insufficient reproducibility of the effect achieved, since the melt mixing of the two-component β-nucleating agent with the polypropylene may lead to varying results, due to the influence of parameters such as melt temperature, shear conditions, compounding time etc.

Efforts accordingly have been made to prepare more reliable systems for achieving β-nucleation, based on dibasic organic acids and Group II metal compounds.

EP 0 682 066 A1 discloses such an attempt for achieving a more reliable β-modification. This document describes that improvements can be achieved by employing a one-component β-nucleating agent, produced by reacting 1 mol of dicarboxylic acid with 1 mol calcium carbonate in an aqueous ethanol containing solution at 60 to 80°C. This reaction yields the calcium salt of the dicarboxylic acid which is obtained in the form of a fine precipitate which can be isolated by filtration. Thereafter, the product is dried and may be used as β-nucleating agent.

The drawback of this one-component β-nucleating agent, namely the calcium salt of the dicarboxylic acid is, on the one hand, the fact that the obtained precipitate contains 1 mol of crystal water which decreases the effect of β-nucleation. The removal of this crystal water is only achievable under severe conditions which, however, increases the cost of the additive, since additional heating steps are required. A further drawback is the fact that the one-component β-nucleating agent is obtained in the form of a fine precipitate, which gives rise to problems during filtration. In particular, fine precipitates are a major drawback when considering the scale up of the synthesis, since fine precipitates will lead to a drastic decrease of filtration efficiency.

It furthermore has to be taken into account that dibasic organic acids are rather expensive raw materials, so that it would be advantageous to reduce the amount of dibasic organic acid required for β-nucleation.

With respect to the other β-nucleating agents, such as the above-mentioned quinacridone pigments, it has to be noted further that same give rise, even when added in low amounts only, to a discoloration of the polypropylene. Even at amounts of below 10 ppm, the color of the polypropylene containing such β-nucleating agents shows a discoloration being discernible with the eye, typically a light red color. This is, of course, a further drawback which decreases the value of the nucleated polypropylene.

## Object of the present invention

In view of the drawbacks identified above, it is the object of the present invention to provide a novel and improved β-nucleating agent, which enables the preparation of polypropylene in the β-modification without giving rise to the drawbacks associated with the conventional β-nucleating agents such as discoloration. Furthermore, the effects as obtained should be reproducible and the use of costly raw materials, such as dibasic organic acids should be reduced as much as possible. The β-nucleating agent furthermore should be obtainable by a process which also enables the preparation of the β-nucleating agent at a larger scale. It furthermore would be advantageous if costly and time consuming further treatments, such as removal of crystal water could be avoided.

## Brief description of the invention

The above objects are solved with a process in accordance with claim 1. Preferred embodiments are disclosed subclaims 2 to 7 as well as in the following specification. The present invention furthermore provides a β-nucleating agent as defined in claims 8 and 11, respectively. Preferred embodiments are again presented in dependent subclaims 9 and 10 as well as 12 and 13, respectively. Finally, the present invention also provides a polypropylene composition, as defined in claim 14.

**Detailed description of the present invention**

As defined in claim 1, the present invention provides a process for preparing a β-nucleating agent, comprising the step of treating a mixture comprising a dibasic organic acid and an oxide, hydroxide or acid salt of a Group II metal at a temperature of above 120°C.

The dibasic acid to be employed in accordance with the present invention may be selected among dibasic organic acids comprising four or more carbon atoms. Preferred examples of dibasic acids are pimelic acid, suberic acid, azelaic acid, o-phthalic acid, terephthalic acid as well as isophthalic acid. These acids may be used singly or in any desired admixture. Preferred are in particular pimelic acid, suberic acid as well as azelaic acid.

The oxide, hydroxide or acid salt of a Group II metal to be employed in accordance with the present invention typically is a compound comprising calcium, magnesium, strontium or barium or mixtures thereof. Preferred are in particular calcium compounds. Suitable examples include calcium hydroxide as well as calcium carbonate, as well as magnesium carbonate, strontium carbonate and barium carbonate. Preferred in particular is calcium carbonate.

The dibasic organic acid and the compound of a Group II metal may be employed in any desired ratio. Suitable are mixtures comprising higher or equal molar amounts of the Group II metal compounds than of the dibasic organic acid. A particularly preferred embodiment of the present invention, however, is the use of a mixture comprising a high excess of the Group II metal compound, which is the less expensive component for the preparation of the β-nucleating agent. Suitable ratios of Group II metal compound to dibasic organic acid are as follows:

**Weight ratio:** 10:5-0.1, in particular, 10:3-0.5; more preferably, 10:2-1
**Mole ratio:** 10:5-0.1, in particular, 10:3-0.5; more preferably, 10:2-1.

In the method in accordance with the present invention, it is, in particular, preferred when the two components, prior to the above-identified heat treatment are admixed intensively, preferably using a ball mill, a roll mill or a corresponding device. This premixing typically is carried out at a temperature of from 10 to 40°C, preferably 20 to 25°C.

As identified above, the components for the preparation of the β-nucleating agents are in accordance with the technical teaching of the present invention subjected to a heat treatment at a temperature of 120°C or more, preferably 140°C or more and most preferably 150°C or more. A particular suitable treatment temperature is about 160°C. The heat treatment may be carried out for a period of time suitable for giving rise to the desired one-component β-nucleating agent in accordance with the present invention and typical examples of treatment times are 30 minutes or longer, more preferably 1 hour or longer, and in particular 1.5 hours or longer, such as about 2 hours. This heat treatment is typically carried out at ambient pressure, either in the presence of air or in the presence of an inert gas, such as nitrogen.

The heat treatment may be carried out in any desired and suitable device, including stirred vessels and ball mills as well as fluidized bed reactors. In particular, the use of fluidized bed reactors is preferred in accordance with the present invention. These devices, which are well known to the skilled person, enable an intimate mixing of the two components and further allows for a continuous process, a further advantage in particular regarding the scale-up of the process in accordance with the present invention.

The heat treatment in accordance with the present invention is carried out in the absence of solvents or other liquid reaction media. The heat treatment, according to the results as illustrated in the following examples, gives rise to a solid-state reaction between the dibasic acid and the Group II metal compound, yielding the corresponding salt of the dibasic acid and, depending upon the ration of starting compounds, residual Group II metal compound. The present invention accordingly enables the preparation of pure Group II metal salt of the dibasic acid, when employing an equimolar mixture, while at the same time, also enabling the preparation of any desired mixture of salt of the dibasic acid and, depending upon the ration of starting compounds, residual Group II metal compound. Surprising in the context of the present invention is on the one hand the fact that the heat treatment in accordance with the present invention gives rise to a solid state reaction, while on the other hand even the not equimolar mixtures yield, after heat treatment, a highly efficient β-nucleating agent, although only low contents of dibasic acid salt are present.

After the heat treatment, the obtained one-component β-nucleating agent may be subjected to further post treatments, in particular after having been cooled down to room temperature. In this respect, it is in particular preferred to subject the obtained one-component β-nucleating agent to a further milling treatment to obtain a fine powder having a weight average particle size of from 1 to 10 μm, preferably 2 to 7 μm, and in particular 3 to 5 μm.

The obtained β-nucleating agent is a one-component β-nucleating agent comprising the Group II metal compound as well as the dibasic organic acid in reacted form. Namely, the dibasic organic acid surprisingly reacts during the heat treatment with the Group II metal compound forming a one-component β-nucleating agent comprising, depending on the compositional ratio, Group II metal compound and the Group II metal salt of the dibasic acid. Free dibasic acid typically is no longer contained in the one-component β-nucleating agent after heat treatment.

In view of the fact that the process for preparing the one-component β-nucleating agent does not involve any reaction steps in aqueous solutions or other liquid reaction media, the drawback of obtaining products comprising crystal water as identified above in connection with the prior art can be avoided. Since the process of the present invention is a solid state reaction time and cost intensive filtering processes can be avoided. Furthermore, depending on the compositional ratio, it is possible to obtain one-component β-nucleating agents comprising only a minor amount of Group II metal salt of the dibasic organic acid in admixture with remaining amounts of Group II metal compound. However, as will be explained further below, even these one-component β-nucleating agents, which can be considered as small particles of Group II metal compound being modified at the surface thereof with the Group II metal salt of the dibasic organic acid, achieve a reproducible and highly efficient β-nucleation in polypropylene compositions, so that it is possible to obtain the desired β-modification of polypropylene with lower amounts of dibasic organic acid required, compared to the prior art β-nucleating agents.

The process in accordance with the present invention furthermore is reliable, simple to carry out and allows a scaling-up by using suitably adapted standard devices, such as fluidized bed reactors. Taking into account that highly efficient and reliable β-nucleating agents may be produced using the present invention it is readily apparent that the present invention adds a significant improvement to the art. Surprising in this respect is in particular the fact that a solid-state reaction is achieved, since previously the salt of the dibasic acid could only be prepared in solution using water as solvent, while attempts to use other polar solvents, such as ethanol, did not give rise to any reaction.

The β-nucleating agent obtained in accordance with the present invention may be used in particulate form, preferably as fine powder, when adding the β-nucleating agent to the polypropylene to be nucleated, but the present invention also envisages the use of the β-nucleating agent in the form of a master-batch, i.e. in the form of a compound comprising a polymeric matrix, preferably a polypropylene, and a rather high concentration of the β-nucleating agent.

The β-nucleating agent obtained by the present invention is used in polypropylene compositions in amounts of from 0.001 to 5 wt.-%, preferably 0.01 to 2 wt.-%, such as from 0.05 to 1 wt.-% (calculated on the basis of the polypropylene content). Thereby a degree of β-modification in the polypropylene of up to 80% or more as determined by DSC can be achieved, even in the presence of other additives, such as fillers, stabilizers, lubricants etc. The amount of β-modification may also be expressed as k value according to Turner-Jones, and the present invention achieves k values of up to 0.94 or more (such as 0.97). Regarding the k value according to Turner-Jones and the calculation thereof reference is made to the corresponding description in EP 0 682 066 A1, incorporated herein by reference.

The polypropylene to which the β-nucleating agent in accordance with the present invention may be added may be a homopolymer as well as a copolymer, including random copolymers as well as block copolymers. The polypropylenes are typically stereoregular polypropylenes, such as isotactic polypropylenes as well as elastomeric polypropylenes having a degree of stereo regularity of preferably 80% or more. Stereoregularity is preferably determined by $^{13}$C-NMR spectroscopy in solution as described e.g. by Busico et al. in Macromolecules 28 (1995) 1887-1892, taking the isotactic pentad regularity (mmmm) as measure of stereoregularity.

In accordance with a further aspect of the present invention, the present invention provides a novel one-component β-nucleating agent, obtainable by a process as identified in the present application.

Preferred embodiments as described above in connection with the process of the present invention likewise also apply with respect to the one-component β-nucleating agent in accordance with the present invention.

Furthermore, the present invention provides a novel β-nucleating agent comprising the solid phase reaction product of a Group II metal compounds selected among oxides, hydroxyls and acid salts, with a dibasic organic acid, wherein the β-nucleating agent comprises particles of Group II metal compound as identified above modified at the surface with a Group II metal salt of a dibasic organic acid.

Preferred embodiments as identified above in connection with the process in accordance with the present invention likewise also apply with respect to the β-nucleating agent defined herein.

In particular the β-nucleating agent comprises a ratio of Group II metal compound to dibasic organic acid as follows:

**Weight ratio:** 10:5-0.1, in particular, 10:3-0.5; more preferably, 10:2-1
**Mole ratio:** 10:5-0.1, in particular, 10:3-0.5; more preferably, 10:2-1.

A particularly preferred β-nucleating agent comprises calcium carbonate and the calcium salt of a dibasic acid, preferably pimelic acid, on the surface of the individual calcium carbonate particles. This structure is the result of the solid state reaction yielding the β-nucleating agent of the present invention. This structure and composition can be confirmed by IR-spectroscopy as well as by microscopic techniques enabling the evaluation of surface portions of individual particles.

Such β-nucleating agents, in particular the embodiments prepared using a large excess of Group II metal compound, do give rise to fully satisfactory and reproducible contents of the desired β-modification in polypropylene compositions while reducing drastically the required amount of dibasic acid needed for the β-nucleation. This is a vast improvement compared with the prior art, yielding either not well reproducible β-nucleation or requiring higher amounts of the costly dibasic acid.

Finally the present invention provides a polypropylene composition, comprising a polypropylene and a β-nucleating agent as described herein. The polypropylene may be any kind of polypropylene, as outlined above, including in particular isotactic polypropylenes, such as homopolymers and copolymers, typically copolymers comprising only small amounts of comonomers, such as copolymers described in the art as random copolymers. The composition as provided by the present invention comprises the β-nucleating agent as described herein in amounts yielding the desired content of β-modification, typically of from 0.001 to 5 wt.-%, preferable 0.01 to 2 wt.-%, and in embodiments 0.05 to 1 wt.-%, based on the content of polypropylene. Other usual additives and fillers may be present as well in typical amounts. The polypropylene composition in accordance with the present invention typically displays a k value according to Turner-Jones of above 0.5, and the resent invention enables the provision of polypropylene compositions showing k values of as much as 0.85 or more. This corresponds to a content of β-modification as determined by DSC of up to 80% or more.

The following examples illustrate the present invention further:

## Examples

Mixtures 1-3 were prepared by intensively mixing of calcium carbonate (supplied by Fluka, Art. No. 21060) and Pimelic acid (supplied by Fluka, Art. No. 80500) at room temperature, then putting the mixtures into a convection oven at 160°C for two hours. After cooling down to room temperature all mixtures were milled to a fine powder with an average particle size of 3-5μm.

| No. | $CaCO_3$ [gram] | Pimelic acid [gram] | Comment | Heat Treatment |
|---|---|---|---|---|
| 1 | 10.00 | 10.00 | wt. by wt. | 2h/160°C |
| 2 | 10.01 | 16.02 | Equimolar mixture | 2h/160°C |
| 3 | 10.00 | 1.00 | wt. by wt. | 2h/160°C |

Another mixture has been prepared in the following way (mixture 4):

16.02 g pimelic acid was dissolved in 100ml of pure ethanol (supplied by Fluka, Art. No. 02883) and heated up to 60°C. then 10,01g $CaCO_3$ (supplied by Fluka, Art. No. 21060) were added and stirred for two hours. No reaction to Ca-Pimelate at all could be detected, since no development of $CO_2$ was seen. The suspension was filtrated, dried at 110°C and identified by IR-spectroscopy. The precipitate could be identified as pure $CaCO_3$ but no Ca-Pimelate could be identified.

Mixtures 1 to 4 were evaluated regarding β-nucleation in polypropylene:

## Recipes and results:

A polypropylene homopolymer powder (MFR(230/2.16): 0.2g/10min) was mixed with 0.5% pentaerythrityl-tetrakis(3-(3',5'-di-tert. butyl-4-hydroxyphenyl)-propionate (supplied as Irganox 1010, Ciba SC), 0.10% tris (2,4-di-*t*-butylphenyl) phosphite (supplied as lrgafos 168, Ciba SC) and 0.10% Ca-Stearate (supplied as Ca-Stearate S, Faci) and the mixtures 1 to 4 as mentioned above were added in amounts as indicated in the following table and extruded with a Theyson TSE 24 twin screw extruder at a melt temperature of 230°C. The obtained products were evaluated and the results are shown in the following table:

| Nucleating agent | Tm Beta [°C] | Tm alpha [°C] | Hm beta [J/g] | Hm alpha [J/g] | Tk [°C] | Beta-content [%] |
|---|---|---|---|---|---|---|
| no (CE1) | 146.7 | 163.7 | 2.8 | 97.3 | 112.2 | <5 |
| 0.1% mixture 1 | 150.8 | 163.8 | 70.1 | 34.9 | 123.1 | 66.8 |

(continued)

| | | | | | | |
|---|---|---|---|---|---|---|
| 0.2% mixture 1 | 152.1 | 165.3 | 69.3 | 28.6 | 122.8 | 70.8 |
| 0.1% mixture 2 | 151.9 | 168.1 | 79.2 | 24.1 | 121.7 | 76.7 |
| 0.2% mixture 2 | 151.8 | 168.2 | 78.0 | 21.4 | 122.6 | 78.5 |
| 0.1% mixture 3 | 152.0 | 164.9 | 69.5 | 35.7 | 124.2 | 66.1 |
| 0.2% mixture 3 | 152.3 | 165.5 | 61.8 | 37.9 | 124.8 | 62.0 |
| 0.2% mixture 4 (CE2) | 148.2 | 164.8 | 3.1 | 96.9 | 113.7 | <5 |

The β-content was calculated as the ratio between to heat of fusion for the melting peak of the β-phase and the total heat of fusion (β-phase + (β-phase):

$$\beta\text{-content} = H_{\beta\text{-phase}}/(H_{\beta\text{-phase}} + H_{\beta\text{-phase}})$$

An additional calculation of the β-content was done via the Turner-Jones equation (A.Turner-Jones et al., Makromol Chem., 75 (1964) 134).

| Nucleating agent | k-value |
|---|---|
| 0.2% mixture 1 | 0.82 |
| 0.2% mixture 2 | 0.71 |
| 0.2% mixture 3 | 0.59 |
| 0.2% mixture 4 | 0.04 |

The β-content of all mixtures containing one of the mixture 1-3 is significantly higher than 60% by DSC, which shows a highly efficiency as β-nucleating agent of these mixtures.

The comparative example 1 (CE1) without β-nucleating agents and the comparative example (CE2) with the solution prepared mixture 4 show no significant β-content.

The k-value according to Turner-Jones is significantly higher than 0.5 for all samples prepared using the mixtures of the present invention, which indicates a high content of the β-modification in the samples and hence a high efficiency of the inventive β-nucleating agent.

From the inventive examples it can be clearly seen that a reduction of the relative amount of the dibasic acid from an equimolar ratio (mixture 2) to the very high weight ratio of 10 (mixture 3) does not compromise the efficiency as β-nucleating agent, even at identical concentrations.

Comparative examples 3 to 5 were prepared according to DE 36 10 644 (the German language equivalent to US 5,231,126) by mixing of pimelic acid with $CaCO_3$ and Ca-stearate respectively (1:1 by weight) without any further treatment.

The same polypropylene homopolymer powder (MFR(230/2.16): 0.2g/10min) as used in the previous examples was mixed with 0.5% pentaerythrityl-tetrakis(3-(3',5'-di-tert. butyl-4-hydroxyphenyl)-propionate (supplied as Irganox 1010, Ciba SC), 0.10% tris (2,4-di-t-butylphenyl) phosphite (supplied as Irgafos 168, Ciba SC) and 0.10% Ca-Stearate (supplied as Ca-Stearate S, Faci) and the $CaCO_3$/pimelic acid mixtures prepared according to DE 36 10 644 as mentioned above and extruded with a Theyson TSE 24 twin screw extruder at a melt temperature of 230°C. The compositions and the results of subsequent evaluations of the products obtained are given in the following table:

| Nucleating agent | Tm Beta [°C] | Tm alpha [°C] | Hm beta [J/g] | Hm alpha [J/g] | Tk [°C] | Beta content [%] |
|---|---|---|---|---|---|---|
| 0.1% pimelic acid/CaSt (CE3) | | 165.4 | 1.6 | 106.6 | 117.0 | <2 |
| 0.1% pimelic acid/CaCO$_3$ (CE4) | | 166.9 | 0.0 | 109.4 | 123.8 | 0.0 |
| 0.2% pimelic acid/CaCO$_3$ (CE5) | | 167.5 | 0.0 | 101.5 | 125.5 | 0.0 |

The comparative examples CE3 to CE5 do not show any indication of an increase of the content of the β-modification of polypropylene as measured by DSC.

Comparative example 6:

With BE 50 (commercial PP-homopolymer from Borealis without any nucleating agent, MFR(230/2.16): 0.2g/10min) 2mm thick compression molded plaques were prepared and the k-value according to Turner-Jones was measured. The k-value was <0.01, which indicates basically no presence of the β-modification of PP. This composition corresponds to the one of comparative example 1 given above.

**Claims**

1.  Process for preparing a β-nucleating agent, comprising the step of heat treating a Group II metal compound with a dibasic organic acid at a temperature of 120°C or more.

2.  Process in accordance with claim 1, wherein the Group II metal compound is a calcium compound.

3.  Method in accordance with claim 1 or 2, wherein the dibasic organic acid is selected among pimelic acid, suberic acid, and azelaic acid.

4.  Process in accordance with claim 1, 2 or 3, wherein the heat treatment is carried out at a temperature of 150°C or more.

5.  Process in accordance with any one of claims 1 to 4, wherein heat treatment is carried out in a fluidized bed reactor.

6.  Process in accordance with any one of claims 1 to 5, wherein the heat treatment is carried out for a duration of 1.5 hours or more.

7.  Process in accordance with any one o claims 1 to 6, wherein the dibasic acid is employed in an amount giving rise to a weight ratio or mole ratio of Group II metal compound to dibasic acid of 10:5-0.1.

8.  β-nucleating agent, obtainable by any of the processes according to claims 1 to 7.

9.  β-nucleating agent in accordance with claim 8, wherein the Group II metal compound and the dibasic organic acid are employed in the process according to any one of claims 1 to 6 in an equimolar amount or in a ratio comprising an molar excess of Group II metal compound.

10. β-nucleating agent in accordance with claim 8 or 9, wherein the mole ratio or the weight ratio of Group II metal compound to dibasic organic acid is 10:1.

11. β-nucleating agent, comprising the solid phase reaction product of a Group II metal compound and a dibasic organic acid, wherein the β-nucleating agent comprises particles of Group II metal compound being modified at the surface thereof with a Group II metal salt of a dibasic organic acid.

12. β-nucleating agent in accordance with claim 11, wherein the Group II metal compound is calcium carbonate and the dibasic organic acid is pimelic acid.

**13.** β-nucleating agent in accordance with claim 11 or 12, wherein the mole ratio or the weight ratio of Group II metal compound to Group II metal salt of the dibasic organic acid is 10:1.

**14.** Polypropylene composition, comprising a polypropylene and a β-nucleating agent according to any one of claims 8 to 13 or a β-nucleating agent obtained by a process according to any one of claims 1 to 7.

**European Patent Office**

## EUROPEAN SEARCH REPORT

Application Number

EP 06 02 6328

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 231 126 A (SHI GUAN-YI [CN] ET AL) 27 July 1993 (1993-07-27) * examples 1-5 * * column 2, line 64 - column 3, line 15 * ----- | 1-10,14 | INV. C07C51/41 C07C55/02 C07C55/16 C07C55/18 C08K5/098 C08L23/12 |
| X | WO 02/078924 A (MILLIKEN & CO [US]) 10 October 2002 (2002-10-10) * example 1 * * page 5, line 24 * ----- | 1-10,14 | |
| X,D | EP 0 682 066 A1 (DANUBIA PETROCHEM POLYMERE [AT] BOREALIS AG [AT]) 15 November 1995 (1995-11-15) * page 3, lines 44-47; examples 2-5 * * page 4; tables 1,2 * ----- | 8-10,14 | |

TECHNICAL FIELDS SEARCHED (IPC)

C07C
C08K
C08L

The present search report has been drawn up for all claims

| Place of search | Date of completion of the search | Examiner |
|---|---|---|
| Munich | 22 June 2007 | Panday, Narendra |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent
Office

Application Number

EP 06 02 6328

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☒ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

# EP 1 939 167 A1

The Search Division considers that the present European patent application does not comply with the requirements of unity of invention and relates to several inventions or groups of inventions, namely:

1. claims: 1-7

   Process for preparing beta-nucleating agent
   ---

2. claims: 8-13

   The beta-nucleating agent itself
   ---

3. claim: 14

   A polypropylene composition comprising the beta-nucleating agent
   ---

## ANNEX TO THE EUROPEAN SEARCH REPORT
## ON EUROPEAN PATENT APPLICATION NO.

EP 06 02 6328

This annex lists the patent family members relating to the patent documents cited in the above-mentioned European search report.
The members are as contained in the European Patent Office EDP file on
The European Patent Office is in no way liable for these particulars which are merely given for the purpose of information.

22-06-2007

| Patent document cited in search report | | Publication date | Patent family member(s) | | Publication date |
|---|---|---|---|---|---|
| US 5231126 | A | 27-07-1993 | NONE | | |
| WO 02078924 | A | 10-10-2002 | AT | 349308 T | 15-01-2007 |
| | | | BR | 0208426 A | 06-07-2004 |
| | | | CN | 1500117 A | 26-05-2004 |
| | | | EP | 1379368 A2 | 14-01-2004 |
| | | | JP | 2004525227 T | 19-08-2004 |
| | | | RU | 2296115 C2 | 27-03-2007 |
| | | | US | 2003149150 A1 | 07-08-2003 |
| | | | US | 2003027908 A1 | 06-02-2003 |
| | | | US | 2004220311 A1 | 04-11-2004 |
| EP 0682066 | A1 | 15-11-1995 | AT | 404252 B | 27-10-1998 |
| | | | AT | 99494 A | 15-02-1998 |
| | | | CZ | 9501233 A3 | 15-11-1995 |
| | | | DE | 59506332 D1 | 12-08-1999 |
| | | | DK | 682066 T3 | 22-11-1999 |
| | | | ES | 2133610 T3 | 16-09-1999 |
| | | | HR | 950263 A2 | 31-10-1997 |
| | | | HU | 74309 A2 | 30-12-1996 |
| | | | JP | 3509997 B2 | 22-03-2004 |
| | | | JP | 8048828 A | 20-02-1996 |
| | | | SK | 62295 A3 | 06-12-1995 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0177961 A2
- US 5231126 A
- EP 0682066 A1
- DE 3610644

### Non-patent literature cited in the description

- **BUSICO et al.** *Macromolecules,* 1995, vol. 28, 1887-1892